(19) Europäisches Patentamt
European Patent Office
Office européen des brevets

(11) EP 4 464 690 A1

(12) EUROPEAN PATENT APPLICATION
published in accordance with Art. 153(4) EPC

(43) Date of publication:
**20.11.2024 Bulletin 2024/47**

(21) Application number: **23740015.5**

(22) Date of filing: **10.01.2023**

(51) International Patent Classification (IPC):
***C07C 271/28*** (2006.01) ***C07C 269/04*** (2006.01)
***C07C 269/06*** (2006.01) ***A61K 31/27*** (2006.01)
***A61K 31/277*** (2006.01) ***A61P 19/00*** (2006.01)
***A61P 1/14*** (2006.01) ***A61P 13/10*** (2006.01)
***A61P 9/12*** (2006.01) ***A61P 11/00*** (2006.01)
***A61P 11/06*** (2006.01) ***A61P 15/00*** (2006.01)
***A61P 17/04*** (2006.01) ***A61P 27/16*** (2006.01)
***A61P 25/00*** (2006.01)

(52) Cooperative Patent Classification (CPC):
**A61P 1/00; A61K 31/27; A61K 31/277; A61P 11/00; A61P 13/10; A61P 15/00; A61P 17/04; A61P 27/16; C07C 271/28;** Y02P 20/55

(86) International application number:
**PCT/CN2023/071587**

(87) International publication number:
**WO 2023/134673 (20.07.2023 Gazette 2023/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.01.2022 CN 202210041774**

(71) Applicant: **Shanghai Institute of Materia Medica, Chinese Academy of Sciences
Pudong, Shanghai 201203 (CN)**

(72) Inventors:
- **NAN, Fajun**
  **Shanghai 201203 (CN)**
- **GAO, Zhaobing**
  **Shanghai 201203 (CN)**
- **DONG, Wuheng**
  **Shanghai 201203 (CN)**
- **ZHENG, Yueming**
  **Shanghai 201203 (CN)**
- **WANG, Jintao**
  **Shanghai 201203 (CN)**
- **ZHOU, Xiaoyu**
  **Shanghai 201203 (CN)**
- **LIU, Huanan**
  **Shanghai 201203 (CN)**
- **XU, Haiyan**
  **Shanghai 201203 (CN)**

(74) Representative: **Grünecker Patent- und Rechtsanwälte
PartG mbB
Leopoldstraße 4
80802 München (DE)**

# (54) HIGH-SELECTIVITY KCNQ4 POTASSIUM CHANNEL AGONIST, PREPARATION METHOD THEREFOR AND USE THEREOF

(57) Disclosed are a high-selectivity KCNQ4 potassium channel agonist of formula I, a preparation method therefor and the use thereof. The compound further improves the KCNQ4 agonistic activity, and is still free of KCNQ2 agonistic activity, thus having excellent KCNQ4/KCNQ2 selectivity. The high-selectivity KCNQ4 agonist overcomes the defect of poor selectivity of existing potassium channel agonists, the activity is relatively increased, and the toxicity is remarkably reduced. The agonist also has a simpler structure and lower product cost, thereby having a better development prospect.



Processed by Luminess, 75001 PARIS (FR)

(Cont. next page)

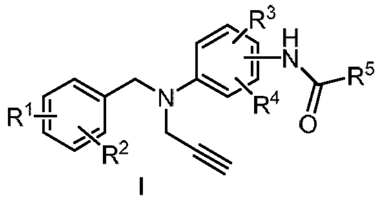

I

## Description

### TECHNICAL FIELD

The present invention relates to the technical field of agonist synthesis, in particular to a new highly selective KCNQ4 potassium channel agonist, its preparation method therefor and a use thereof in preparation of a medicament for treating conditions associated with smooth muscle or skeletal muscle dysfunction such as visceral pain, indigestion, irritable bowel syndrome, overactive bladder syndrome, hypertension, pulmonary hypertension, coronary artery disease, cerebral vasospasm, asthma, chronic obstructive pulmonary disease, prenatal labor pains, pruritus, sexual dysfunction and deafness.

### BACKGROUND OF THE ART

Voltage-gated potassium channels (Kv channels) are currently the most discovered ion channel family, with a total of 12 members (Kv1.X-Kv12.X). KCNQ channels are the 7$^{th}$ subfamily of Kv channels (Kv7), including five subtypes, namely, KCNQ1-KCNQ5. KCNQ1 is mainly distributed in the cardiac system, and together with the auxiliary subunit KCNQ1, it forms the $I_{Ks}$ current in the myocardial action potential and is responsible for the repolarization of myocardial action potential. KCNQ2-KCNQ5 are mainly distributed in the central and peripheral nervous systems, also known as neural KCNQ channels, regulating membrane potential levels and neural excitability (Brown et al., Br J Pharmacol. 2009, 156(8): 1185-1195); KCNQ4 and KCNQ5 channels are also distributed in smooth and skeletal muscle, regulating muscle contraction and relaxation by affecting membrane potential levels (Stott et al., Drug Discov Today. 2014, 19(4): 413-424). Human genetic studies have shown that KCNQ gene mutations can cause cardiac arrhythmias, epilepsy and congenital deafness (Jentsch, Nat Rev Neurosci. 2000, 1(1): 21-30). The activation threshold of KCNQ channel current is low (it can be opened at a subthreshold potential of about -60 mV), the activation process thereof is slow, and it will not become inactivated after activation. Therefore, it plays a basic role in the electrical excitability of excitable cells. Expression distribution, genetic and pharmacological studies all support that enhanced non-selective KCNQ2-KCNQ5 channel agonists can be used as drugs for the treatment, relief or control of somatic pain, visceral pain, inflammatory pain, neuropathic pain and other diseases (Du et al., Br J Pharmacol. 2018,175(12):2158-2172). The non-selective KCNQ channel agonist flupirtine has been approved in Europe since 1984 for the treatment of acute or chronic pain (Szelenyi, Inflamm Res. 2013, 62(3): 251-258).

KCNQ4 channels are specifically highly expressed in a variety of internal organs and tissues, and are basically not expressed in the central nervous system. This specific tissue distribution provides a structural basis for targeted regulation of KCNQ4 channels and further for the treatment of related diseases. Most of the visceral tissues that have been detected show a trend of high expression of KCNQ4 subtype. For example, the expression of KCNQ channels in various vascular arterial smooth muscles of mice was analyzed and it was found that the expression levels of the five subtypes are: KCNQ4>KCNQ5>>KCNQ1, and the expression levels of KCNQ2 and KCNQ3 were extremely low (Yeung et al., Br J Pharmacol. 2007, 151(6):758-770). The expression of KCNQ4 is the highest in different parts of the gastrointestinal smooth muscle, including the colon, jejunum, gastric antrum, fundus, etc. (Ipavec et al., Pharmacol Res. 2011, 64(4):397-409). KCNQ4 channels are abundantly expressed in rodent respiratory tracheal smooth muscle and uterine smooth muscle (Evseev et al., Front Physiol. 2013, 4:277). Meanwhile, the results of KCNQ distribution study in human visceral tissues also show high expression of KCNQ4 (Ng et al., Br J Pharmacol. 2011, 162(1):42-53). Specific targeting of KCNQ4 can avoid the side effects of activating other subtypes, for example, long QT syndrome may be caused by inhibiting KCNQ1 channels and KCNQ1-mediated $I_{Ks}$ currents (Terrenoire et al., Circ Res. 2005, 96(5):e25-34), and can also effectively avoid side effects such as sedation and drowsiness caused by enhancing KCNQ2 and KCNQ3 channels in the central nervous system (Orhan et al., Expert Opin Pharmacother. 2012, 13(12): 1807-16). Existing KCNQ agonists generally suffer from poor selectivity, especially for KCNQ2, KCNQ4 and KCNQ5 channels. All discovered KCNQ2 agonists affect KCNQ4 channels simultaneously. Among them, only RL648_81 (ethyl (2-amino-3-fluoro-4-((4-(trifluoromethyl)benzyl)amino)phenyl)amino formate) just enhances the KCNQ2 channel without affecting the KCNQ4 channel (Kumar et al., Mol Pharmacol. 2016, 89(6):667-677). The selectivity of the existing KCNQ4 agonists is not ideal. Except for the vasodilator fasudil reported by Xuan Zhang et al. in November 2016, which has a relatively selective agonistic effect on the KCNQ4/KCNQ5 channels, there is no other KCNQ4 agonist displaying selectivity for KCNQ4 (Zhang et al., Br J Pharmacol. 2016,173(24):3480-3491).

In view of the important role of KCNQ4 channel in nervous and smooth muscle systems, KCNQ4 channel agonists can be used for, but are not limited to, the treatment of the following diseases, including visceral pain, indigestion, irritable bowel syndrome, overactive bladder syndrome, hypertension, pulmonary hypertension, coronary artery disease, cerebral vasospasm, asthma, chronic obstructive pulmonary disease, prenatal labor pain, pruritus, sexual dysfunction, deafness (Haick et al., Pharmacol Ther. 2016, 165:14-25; Barrese et al., Annu Rev Pharmacol Toxicol. 2018, 58:625-648).

The reported KCNQ potassium channel agonists are mainly as follows:

1) Some compounds with the following structure are disclosed in Patent No. US5384330:

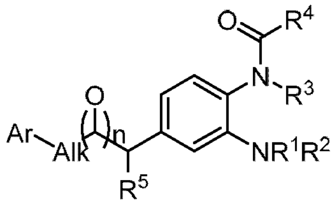

,

which is characterized by containing a benzene ring substituted with two amine groups at ortho positions.

2) a KCNQ potassium channel agonist with the following structure is described in Patent publication No. WO2005/087754:

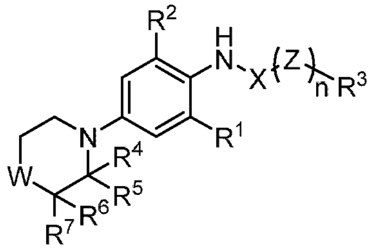

,

which is characterized by containing a benzene ring substituted by two amine groups at para positions, of which one of the nitrogens is in a saturated ring (it may also be a heterocyclic ring, in which case W=O), and the other nitrogen is adjacent to $R^1$ and $R^2$.

3) A compound with the following structure is described in Patent publication No. WO2008024398:

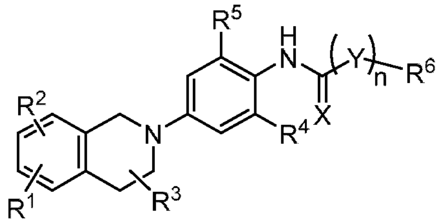

The structure of this compound is similar to that of the compound in patent WO2005/087754, except that a fused benzene ring structural unit is introduced into the nitrogen heterocyclic hydrocarbon.

The most representative KCNQ potassium channel agonist in clinical practice is Retigabine (RTG), an anti-epileptic drug developed by GSK and launched in 2011. Its structure is:

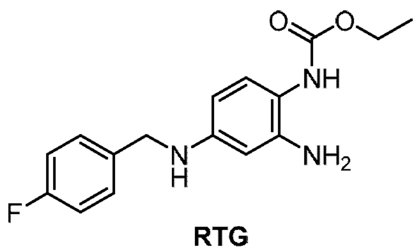

**RTG**

Retigabine is the first KCNQ potassium channel agonist that has been systematically studied. It can activate KCNQ2-5 and is mainly used for the treatment of adult patients with partial-onset epilepsy.

Retigabine contains an electron-rich benzene ring substituted by three amine groups in the structure, which makes it particularly susceptible to oxidation and deterioration during synthesis and storage. Meanwhile, retigabine results in many adverse reactions in clinical applications, including dizziness, drowsiness, fatigue, confusion, tremor, poor coordination, diplopia, blurred vision, attention disorder, memory loss, ataxia, aphasia, dysphonia, balance disorder, increased appetite, hallucinations, myoclonus, peripheral edema, hypokinesia, dry mouth, dysphagia, etc. Abnormal

urination is also a common side effect of retigabine, including bladder swelling, bladder wall thickening, and urinary retention. On April 26, 2013, the FDA Drug Safety Committee disclosed that retigabine may also cause some pigment reactions during clinical application, including blue skin, retinal pigment changes, etc., but its specific mechanism of the action is not yet clear, and it is recommended that all patients taking this drug should undergo regular eye examinations (S. Jankovic et al., Expert Opinion on Drug Discovery, 2013, 8(11), 1-9; F. Rode et al., European Journal of Pharmacology, 2010, 638, 121-127) .

In previous study (WO2013060097) of the inventors, a KCNQ potassium channel agonist with the following structure is disclosed:

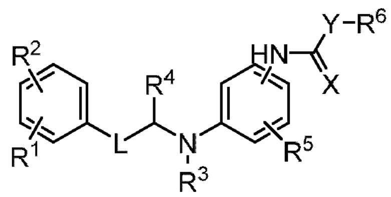

wherein, when $R^3$ is allyl or propargyl, the compound not only maintains KCNQ potassium channel agonistic activity that is equivalent to or better than retigabine, but also has significant anti-epileptic effects in vivo, and the protective effect is equivalent to retigabine; the preliminary pharmacokinetic studies in mice have shown that this compound has superior brain exposure compared with retigabine. However, it is found from further safety evaluation studies that the compounds disclosed in WO2013060097 are highly neurotoxic.

In the subsequent study (CN105017085A) of the inventors, a KCNQ potassium channel agonist with the following structure is disclosed:

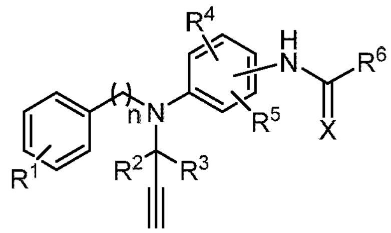

wherein, when the introduced substituents $R^4$ and $R^5$ are alkyl groups in which methyl is a representative group (for example HN37), the resulting compound not only has stable physical properties and is well absorbed by brain tissue, but also has greatly improved KCNQ2 agonistic activity. However, as KCNQ2 agonistic activity increases, the corresponding KCNQ4 agonistic activity also increases accordingly.

When reviewing the previously reported work, the inventors of the present invention found that existing KCNQ agonists generally suffer from poor selectivity, especially for KCNQ2 and KCNQ4 channels. All discovered KCNQ2 agonists affect KCNQ4 channels simultaneously, and among them, only RL648_81 (ethyl (2-amino-3-fluoro-4-((4-(trifluoromethyl)benzyl)amino)phenyl)amino formate) just enhances the KCNQ2 channel without affecting the KCNQ4 channel (Kumar et al., Mol Pharmacol. 2016;89(6):667-77). Currently, the selectivity of existing KCNQ4 agonists is not ideal. Except for the vasodilator fasudil reported by Xuan Zhang et al. in November 2016, which has a relatively selective agonistic effect on the KCNQ4/KCNQ5 channels, there is no other KCNQ4 agonist which was reported to have selectivity for KCNQ4 (Zhang et al., Br J Pharmacol. 2016,173(24):3480-3491). Considering the above-mentioned shortcomings of poor selectivity of existing KCNQ potassium channel agonists, it is necessary to develop new potassium channel agonists, which has better selectivity for KCNQ4 and is used for a new drug for visceral pain, indigestion, irritable bowel syndrome, overactive bladder syndrome, hypertension, pulmonary hypertension, coronary artery disease, cerebral vasospasm, asthma, chronic obstructive pulmonary disease, prenatal labor pain, pruritus, sexual dysfunction, deafness and other diseases.

## SUMMARY OF THE INVENTION

One of the objects of the present invention is to provide a new compound that can be used as a highly selective KCNQ4 potassium channel agonist.

The second object of the present invention is to provide a preparation method of the above-mentioned compound.

The third object of the present invention is to provide a pharmaceutical composition, which comprises the

compound or the pharmaceutically acceptable salt thereof as an active ingredient and optionally, a pharmaceutically acceptable excipient.

The fourth object of the present invention is to provide a use of the above-mentioned compound or the pharmaceutically acceptable salt thereof or the composition containing the same in preparation of a KCNQ4 potassium channel agonist.

The fifth object of the present invention is to provide a use of the above-mentioned compound or the pharmaceutically acceptable salt thereof or the composition containing the same in preparation of a medicament for treating a disease associated with smooth muscle or skeletal muscle or the like. In one aspect, the invention provides a compound of formula I or a pharmaceutically acceptable salt thereof,

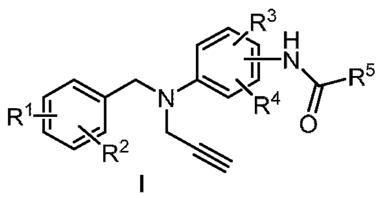

I

wherein:

$R^1$ and $R^2$ are each independently selected from the group consisting of hydrogen, halogen, $C_1$-$C_6$ alkoxy, halogenated $C_1$-$C_6$ alkoxy, $C_1$-$C_6$ alkylthio, $C_4$-$C_6$ tert-alkyl, halogenated $C_1$-$C_6$ alkyl, nitro and cyano; preferably, $R^1$ and $R^2$ are each independently selected from the group consisting of halogen, tert-butyl, nitro, cyano and trifluoromethyl;

$R^3$ and $R^4$ are each independently selected from the group consisting of hydrogen, halogen, and $C_1$-$C_6$ alkyl; preferably, $R^3$ and $R^4$ are each independently hydrogen;

$R^5$ is selected from the group consisting of $C_1$-$C_6$ alkyl, $C_1$-$C_3$ alkoxy, $C_4$-$C_{14}$ tert-alkoxy, phenoxy, and benzyloxy; preferably, $R^5$ is selected from the group consisting of $C_1$-$C_3$ alkoxy, $C_4$-$C_6$ tert-alkoxy, phenoxy, and benzyloxy; more preferably, $R^5$ is selected from the group consisting of methoxy, ethoxy, isopropoxy, tert-butoxy, phenoxy, and benzyloxy; $R^5$ is most preferably tert-butoxy.

In some embodiments, the compound of Formula I is a compound of Formula II below:

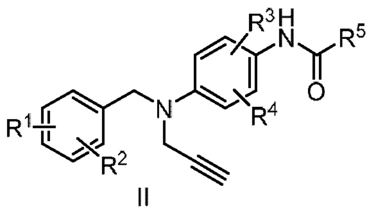

II

wherein the definitions of $R^1$, $R^2$, $R^3$, $R^4$ and $R^5$ are the same as those mentioned above.

In some embodiments, the compound of Formula I is a compound of Formula III below:

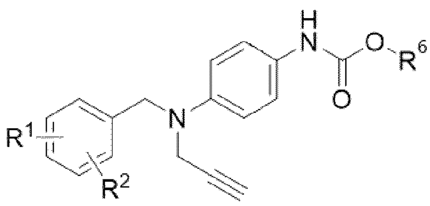

III

wherein the definitions of $R^1$ and $R^2$ are the same as those mentioned above;

$R^6$ is $C_4$-$C_6$ tert-alkyl; and $R^6$ is preferably tert-butyl.

In some embodiments, the compound of formula I is a compound of formula c below:

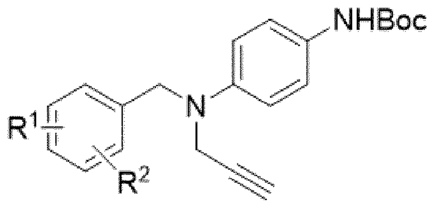

c

wherein the definitions of $R^1$ and $R^2$ are the same as those mentioned above.

In some preferable embodiments, the compound of formula I is selected from the following compounds:

| Compound Number | Compound Structure |
|---|---|
| K31 | 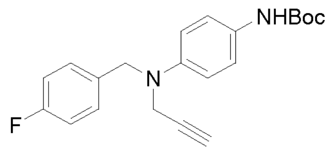 |
| K32 | 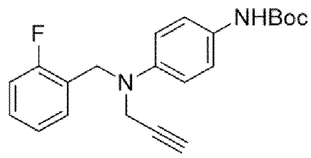 |
| K33 | 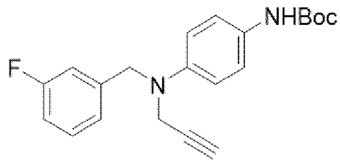 |
| K34 | 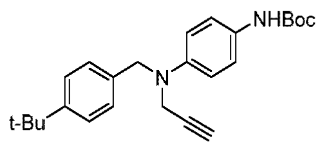 |
| K35 | 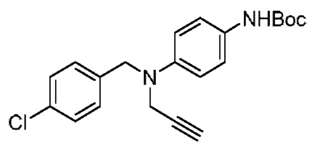 |
| K36 | 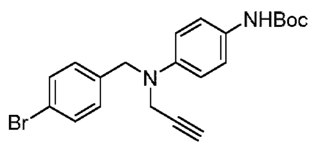 |
| K37 | 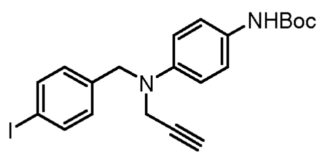 |
| K38 | 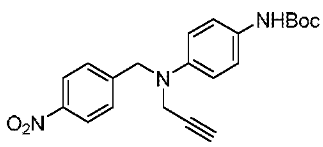 |

(continued)

| Compound Number | Compound Structure |
|---|---|
| K39 | 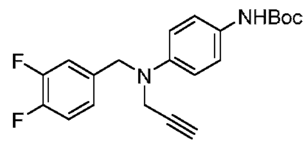 |
| K40 | 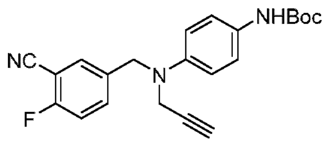 |
| K41 | 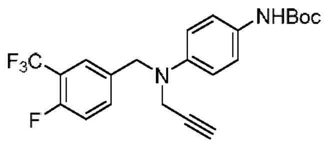 |
| K42 | 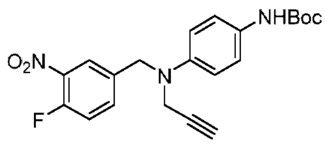 |
| K43 | 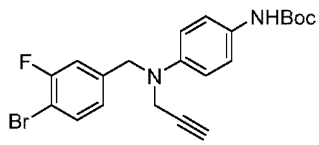 |

The terms used in this invention are defined as follows:
The "halogen" may be fluorine, chlorine, bromine or iodine.

The "$C_1$-$C_6$ alkyl" refers to a chain alkyl with 1-6 carbon atoms; and specific examples thereof may include but are not limited to methyl, ethyl, propyl, n-propyl, isopropyl, butyl, n-butyl, isobutyl, tert-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, and the like.

The "halogenated $C_1$-$C_6$ alkyl" refers to a $C_1$-$C_6$ heterochain alkyl in which at least one hydrogen is substituted with halogen; and specific examples thereof include trifluoromethyl, and the like.

The "$C_4$-$C_6$ tert-alkyl" refers to an alkyl with 4-6 carbon atoms and two branches; and specific examples thereof may include tert-butyl, tert-pentyl, etc.

The "$C_1$-$C_3$ alkoxy" refers to the RO- group, where R is a $C_1$-$C_{14}$ alkyl as described above; and specific examples of the alkoxy include methoxy, ethoxy, n-propoxy, isopropoxy, and the like.

The "$C_4$-$C_6$ tert-alkoxy" refers to an alkoxy group with 4-6 carbon atoms and two branches; and specific examples thereof may include tert-butoxy, tert-pentyloxy, etc. The definition of $C_4$-$C_{14}$ tert-alkoxy can be deduced by analogy.

The pharmaceutically acceptable salt of the compound described in the present invention can be a salt formed by the above-mentioned compound and an acid, and the acid is selected from the group consisting of maleic acid, succinic acid, citric acid, tartaric acid, fumaric acid, formic acid, acetic acid, propionic acid, malonic acid, oxalic acid, benzoic acid, phthalic acid, methanesulfonic acid, benzenesulfonic acid, toluenesulfonic acid, naphthalenesulfonic acid, 1,5-naphthalenedisulfonic acid, camphoric acid, camphorsulfonic acid, salicyl acid, acetylsalicylic acid, aspartic acid, glutamic acid, lactic acid, gluconic acid, vscorbic acid, gallic acid, mandelic acid, malic acid, sorbic acid, trifluoroacetic acid, taurine, homotaurine, 2-hydroxyethanesulfonic acid, cinnamic acid, hydrochloric acid, hydrobromic acid, hydroiodic acid, sulfuric acid, nitric acid, phosphoric acid and perchloric acid.

The compound and the pharmaceutically acceptable salt thereof involved in this application may have an isomer or a racemate, such as an optical isomer (including a diastereomer and an enantiomer), an atropisomer, a geometric isomer (cis-trans isomers), a conformational isomer, a tautomer and mixtures thereof, etc., but are not limited to these. These isomers are also included in the scope defined by the claims of the present invention.

In previous studies, the inventors of the present invention found, when different substituents are introduced into the benzene ring connected to the amino group, especially when the introduced substituents are alkyl group represented

by two methyl groups (such as HN37, CN105017085A) the agonistic activity of KCNQ2 can be greatly enhanced. In the present invention, the inventors further studied and found that, the agonistic activity of KCNQ4 can be greatly enhanced when the substituent on the terminal nitrogen atom was replaced from methyl formate to tert-butyl formate on the basis of retaining the overall skeleton of HN37. Further research showed that, when the substituent on the terminal nitrogen atom was kept as tert-butyl formate and all the substituents on the benzene ring connected to the amino group were removed, the obtained compound K31 not only still had good KCNQ4 agonistic activity, but also lost KCNQ2 agonistic activity, that is to say, it has very good selectivity. On this basis, when different substituents are then introduced to the benzene ring of the benzyl group connected to the amino group, especially when the introduced substituent is an electron-rich substituent represented by tert-butyl group, it is found that not only was the KCNQ4 agonistic activity of the obtained compound further improved, the KCNQ2 agonistic activity was also still lost, and the obtained compound still had good KCNQ4/KCNQ2 selectivity. In summary, the novel selective KCNQ4 agonist provided by the present invention overcomes the shortcomings of poor selectivity of existing potassium channel agonists, has improved activity and significantly reduced toxicity, and has a simpler structure. And the production cost thereof is lower, so it possesses better development prospects.

In another aspect, the present invention provides a preparation method of the compound of formula c, which is accomplished through the following reaction route:

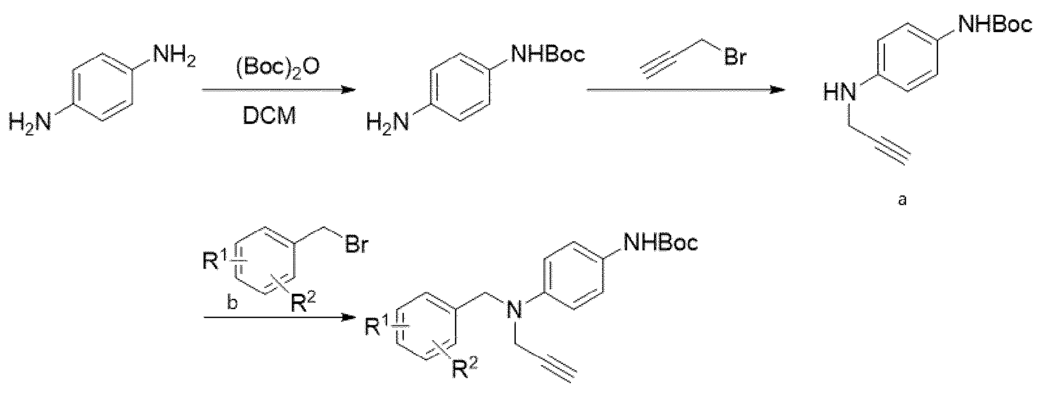


a

c

In the preparation method of the present invention, the definitions of $R^1$ and $R^2$ are the same as those mentioned above.

reacting 1,4-phenylenediamine with di-tert-butyl dicarbonate to produce N-(tert-butoxycarbonyl)-1,4-phenylenediamine; subjecting N-(tert-butoxycarbonyl)-1,4-phenylenediamine and bromopropyne to a substitution reaction to obtain intermediate a; and reacting the intermediate a with compound b to produce compound c (the compound of formula c).

In still another aspect, the present invention provides a pharmaceutical composition comprising the compound or the pharmaceutically acceptable salt thereof according to the present invention and optionally, a pharmaceutically acceptable excipient.

In further another aspect, the present invention provides a use of the compound or the pharmaceutically acceptable salt thereof according to the present invention, or the pharmaceutical composition according to the present invention in preparation of a KCNQ4 potassium channel agonist.

In still another aspect, the present invention provides a use of the compound or the pharmaceutically acceptable salt thereof according to the present invention, or the pharmaceutical composition according to the present invention in preparation of a medicament for treating a disease associated with smooth muscle or skeletal muscle.

In still another aspect, the present invention provides a method for treating a neurological disease, wherein the compound or the pharmaceutically acceptable salt thereof according to the present invention or the pharmaceutical composition according to the present invention is administered to a subject suffering from a disease associated with smooth muscle or skeletal muscle or the like;

The disease associated with smooth muscle or skeletal muscle diseases associated with smooth muscle or skeletal muscle such as visceral pain, indigestion, irritable bowel syndrome, overactive bladder syndrome, hypertension, pulmonary hypertension, coronary artery disease, cerebral vasospasm, asthma, chronic obstructive pulmonary disease, prenatal labor pain, pruritus, sexual dysfunction, deafness, and the like.

The invention has the following beneficial effects:

Compared with existing KCNQ agonists, the compound provided by the invention has greatly improved selectivity for KCNQ4 and KCNQ2. For example, in in vitro electrophysiological experiments, compounds K31-K43 all have good selectivity for KCNQ4 and KCNQ2. They only have KCNQ4 agonistic activity and have no effect on KCNQ2.

Compared with the existing drug retigabine, the compound provided by the present invention has more stable physical properties and is less susceptible to oxidation and deterioration due to the absence of free amine groups in its

structure. It can be shown that the solution of this kind of compound is not easily oxidized and discolored even when exposed to air.

In summary, the compound provided by the present invention overcomes the shortcomings of poor selectivity of existing agonists. It not only has stable physical properties and high activity, but also has good selectivity and greatly reduced toxicity. Therefore, it has a larger therapeutic window and is more effective. It is shown that the compound has good application prospects.

The present invention has been described in detail above, but the above-mentioned embodiments are only illustrative in nature and are not intended to limit the present invention. Furthermore, there is no intention to be bound by any theory presented in the preceding prior art or summary of the invention or in the following examples.

## DETAILED DESCRIPTION OF EXEMPLARY EMBODIMENTS

The present invention will be further described below with reference to the examples. It should be noted that the following examples are provided for illustrative purposes only and do not constitute a limitation on the scope of protection claimed by the present invention.

Unless otherwise specified, the raw materials, reagents, methods, etc. used in the examples are all conventional raw materials, reagents, and methods in this field.

### Preparation Examples of Compounds

In the following preparation examples, nuclear magnetic resonance (NMR) was measured with a Bruker NMR Spectrometer 400M instrument produced by Bruker. NMR calibration: $\delta$H7.26 ppm ($CDCl_3$), 2.50 ppm (DMSO-$d_6$), 3.15 ppm ($CD_3OD$);

Reagents are mainly provided by Shanghai Bide Pharmaceutical Technology Co., Ltd.;
Thin layer chromatography (TLC) silica gel plate is produced by Shandong Yantai Huiyou Silica Gel Development Co., Ltd., model HSGF254;
The compound was purified by normal phase column chromatography, in which silica gel used was produced by Qingdao Marine Chemical Plant Branch in Shandong Province, model zcx-11, 200-300 mesh.

### Preparation Example:

### Preparation Example 1: Synthesis of tert-butyl 4-(N-p-fluorobenzyl-N-propargyl-amino)-phenylaminocarbamate (K31)

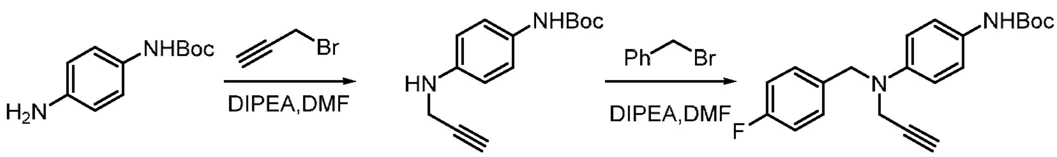


K31-a K31-b K31

To a solution of compound N-(tert-butoxycarbonyl)-1,4-phenylenediamine **K31-a** (1.25 g, 6.0 mmol) in DMF (20 mL), diisopropylethylamine (1.16 g, 9.0 mmol) and propargyl bromide (0.71 g, 6.0 mmol) was added, and the resulting mixture was heated to 65°C for 12 hours. After the reaction was completed, the resultant was cooled to room temperature, and ethyl acetate (50 mL) was added. The organic layer was washed once with water (15 mL) and once with saturated brine (15 mL) respectively, dried over anhydrous sodium sulfate, and concentrated to obtain a crude product. The crude product was purified by a silica gel column chromatography (eluent: petroleum ether/ethyl acetate = 5:1) to obtain a product **K31-b** as a white solid (1.21 g, yield 82%), which was directly used in the next reaction.

To a solution of compound **K31-b** (73.89 mg, 0.3 mmol) in DMF (3 mL), diisopropylethylamine (0.1 mL, 0.6 mmol) and p-fluorobenzyl bromide (68.05 mg, 0.36 mmol) was added. The resulting mixture was heated to 65°C for 12 hours. After the reaction was completed, the resultant was cooled to room temperature, and ethyl acetate (20 mL) was added. The organic layer was washed once with water (5 mL) and once with saturated brine (5 mL) respectively, dried over anhydrous sodium sulfate, and concentrated to obtain a crude product. The crude product was purified by a silica gel column chromatography (eluent: petroleum ether/ethyl acetate = 20:1) to obtain a product **K31** as a white solid (0.95 g, yield 90%).

$^1H$ NMR (400 MHz ,$CDCl_3$): δ 7.30-7.27 (m, 2H), 7.24-7.22 (d, *J*=8.0 Hz, 2H), 7.01 (t, *J* = 8.0 Hz, 2H), 6.86-6.84 (d, *J* = 8.0 Hz, 2H), 6.30 (dr, 1H), 4.43 (s, 2H), 3.93 (s, 2H), 2.22 (t, *J* = 4.0 Hz, 1H), 1.59 (s, 9H).

The following compounds were prepared by using operations similar to Preparation Example 1:

| Compound | Structural Formula | $^1H$ NMR (400 MHz ,$CDCl_3$) data, δ |
|---|---|---|
| K32 | 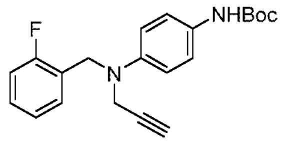 | 7.31 (td, *J* = 7.8, 1.8 Hz, 1H), 7.25-7.21 (m, 3H), 7.08-7.03 (m, 2H), 6.85 (d, *J* = 9.0 Hz, 2H), 6.28 (s, 1H), 4.54 (s, 2H), 4.00 (d, *J* = 2.4 Hz, 2H), 2.22 (t, *J* = 2.0 Hz, 1H), 1.50 (s, 9H). |
| K33 | 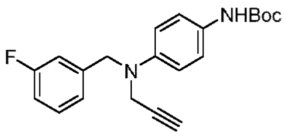 | 7.31-7.26 (m, 1H), 7.22 (*d*, *J* = 8.4 Hz, 2H), 7.10 (d, *J* = 7.6 Hz, 1H), 7.04 (d, *J* = 9.6 Hz, 1H), 6.94 (td, *J* = 8.4, 2.6 Hz, 1H), 6.84 (d, *J* = 9.0 Hz, 2H), 6.29 (s, 1H), 4.47 (s, 2H), 3.97 (d, *J* = 2.4 Hz, 2H), 2.22 (t, *J* = 2.4 Hz, 1H), 1.50 (s, 9H). |
| K34 | 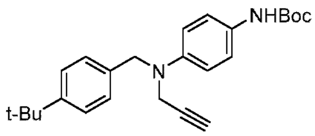 | 7.35 (d, *J* = 8.4 Hz, 2H), 7.25-7.21 (m, 3H), 6.87 (d, *J* = 9.0 Hz, 2H), 6.28 (s, 1H), 4.45 (s, 2H), 3.95 (d, *J* = 2.4 Hz, 2H), 2.20 (t, *J* = 2.4 Hz, 1H), 1.50 (s, 9H), 1.31 (s, 9H). |
| K35 | 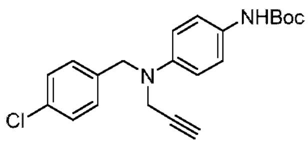 | 7.29 (d, *J* = 8.6 Hz, 2H), 7.26 - 7.20 (m, 3H), 6.84 (d, *J* = 9.0 Hz, 2H), 6.30 (s, 1H), 4.43 (s, 2H), 3.94 (d, *J* = 2.4 Hz, 2H), 2.21 (t, *J* = 2.4 Hz, 1H), 1.50 (s, 9H). |
| K36 | 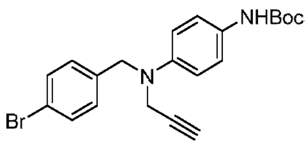 | 7.44 (d, *J* = 8.4 Hz, 2H), 7.24-7.19 (m, 4H), 6.83 (d, *J* = 9.0 Hz, 2H), 6.29 (s, 1H), 4.42 (s, 2H), 3.94 (d, *J* = 2.4 Hz, 2H), 2.21 (t, *J* = 2.4 Hz, 1H), 1.50 (s, 9H). |
| K37 | 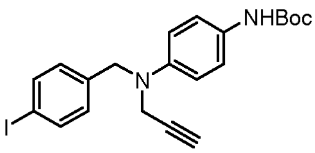 | 7.64 (d, *J* = 8.0 Hz, 2H), 7.22 (d, *J* = 8.4 Hz, 2H), 7.07 (d, *J* = 8.4 Hz, 2H), 6.82 (d, *J* = 9.2 Hz, 2H), 6.30 (s, 1H), 4.41 (s, 2H), 3.94 (d, *J* = 2.4 Hz, 2H), 2.21 (t, *J* = 2.4 Hz, 1H), 1.50 (s, 9H). |
| K38 | 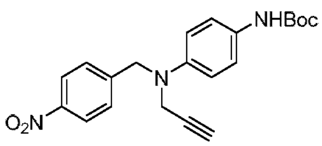 | 8.18 (d, *J* = 8.4 Hz, 2H), 7.49 (d, *J* = 8.4 Hz, 2H), 7.24 (d, *J* = 8.4 Hz, 2H), 6.80 (d, *J* = 9.2 Hz, 2H), 6.37 (s, 1H), 4.57 (s, 2H), 4.00 (d, *J* = 2.4 Hz, 2H), 2.25 (t, *J* = 2.4 Hz, 1H), 1.50 (s, 9H). |
| K39 | 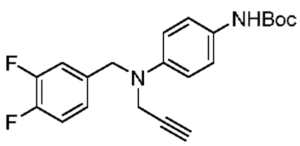 | 7.23 (d, *J* = 8.8 Hz, 2H), 7.18 - 7.00 (m, 3H), 6.83 (d, *J* = 8.8 Hz, 2H), 6.30 (s, 1H), 4.41 (s, 2H), 3.95 (d, *J* = 2.4 Hz, 2H), 2.23 (t, *J* = 2.4 Hz, 1H), 1.50 (s, 9H). |
| K40 | 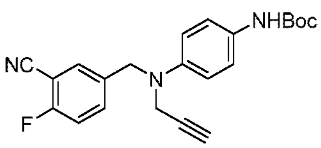 | 7.61 - 7.52 (m, 2H), 7.27 - 7.21 (m, 2H), 7.17 (t, *J* = 8.4 Hz, 1H), 6.82 (d, *J* = 8.8 Hz, 2H), 6.36 (s, 1H), 4.45 (s, 2H), 3.95 (d, *J* = 2.4 Hz, 2H), 2.25 (t, *J* = 2.4 Hz, 1H), 1.50 (s, 9H). |
| K41 | 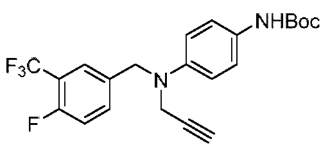 | 7.59 - 7.49 (m, 2H), 7.26-7.23 (m, 2H), 7.15 (t, *J* = 9.2 Hz, 1H), 6.84 (d, *J* =8.8 Hz, 2H), 6.31 (s, 1H), 4.46 (s, 2H), 3.95 (d, *J* = 2.4 Hz, 2H), 2.23 (t, *J* = 2.4 Hz, 1H), 1.50 (s, 9H). |

(continued)

| Compound | Structural Formula | $^1H$ NMR (400 MHz ,$CDCl_3$) data, δ |
|---|---|---|
| K42 | 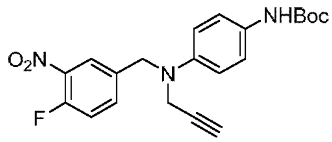 | 8.04 (dd, *J* = 6.8, 2.4 Hz, 1H), 7.62-7.58 (m, 1H), 7.30 - 7.24 (m, 2H), 6.83 (d, *J* = 8.8 Hz, 2H), 6.38 (s, 1H), 4.49 (s, 2H), 3.98 (d, *J* = 2.4 Hz, 2H), 2.26 (t, *J* = 2.4 Hz, 1H), 1.50 (s, 9H). |
| K43 | 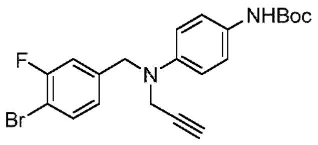 | 7.48 (dd, *J* = 8.0, 6.8 Hz, 1H), 7.23 (d, *J* = 8.4 Hz, 2H), 7.11 (dd, *J* = 9.6, 2.0 Hz, 1H), 7.02 - 6.99 (m, 1H), 6.82 (d, *J* = 8.8 Hz, 2H), 6.30 (s, 1H), 4.42 (s, 2H), 3.96 (d, *J* = 2.4 Hz, 2H), 2.23 (t, *J* = 2.4 Hz, 1H), 1.50 (s, 9H). |

**Electrophysiological Experiment Examples**

**Electrophysiological Experiment Example 1:**

The cell line used in the electrophysiological experiments was the Chinese hamster ovary cell line (CHO-K1); KCNQ cDNA was transformed into *E. coli,* expressed in *E. coli,* and then confirmed by plasmid extraction and sequencing.

1. Cell culture and transfection

CHO-K1 cells (Cell Bank of Chinese Academy of Sciences), medium formula: 50/50 DMEM/F-12 (Gibco), supplemented with 10% fetal bovine serum (FBS) (Gibco, Australia) and 2mM L-glutamine (Invitrogen). Transfection and expression of KCNQ channels: 24 hours before transfection, cells were digested with trypsin (Sigma, China) and spread in a 6-well plate. Lipofectamine2000TM reagent (Invitrogen) was used for transfection, and the experiment was carried out according to the procedures provided. Twenty-four hours after transfection, cells were digested and seeded onto poly-L-lysine (Sigma)-soaked slides. After co-transfected with GFP (green fluorescent protein), the transfected cells were determined under a fluorescence microscope (Olympus). The human KCNQ2 plasmid (NCBI: NM_172107.4) and human KCNQ4 plasmid (NCBI: NM_004700.4) used were synthesized by Beijing Genomics Institute and constructed in the pcDNA3.1(+) vector plasmid. KCNQ2 and KCNQ4 plasmids were confirmed by plasmid extraction and sequencing after transformation into *E. coli* DH5α.

2. Electrophysiological recording in CHO cells:

Whole-cell voltage clamp experiments were carried out at room temperature (23~25°C), where Axopatch-700B amplifier (Molecular Devices, Sunnyvale, CA) and Digidata 1440A (Molecular Devices) as digital-to-analog converter were used, pClamp 10.0 software (Molecular Devices, Sunnyvale, CA) was used for signal acquisition, the signal was filtered at 2 kHz, the sampling frequency was 10 kHz. Series resistance compensation was used and set to 60%. Pipettes were pulled from Borosilicate glass capillaries (World Precision Instruments, Sarasota, FL), and the resistance of the pipettes filled with an intracellular fluid was 3-5 MΩ. The perfusion drug delivery system was BPS-8 (ALA Scientific Instruments, Westburg, NY) with a speed of approximately 1 mL/min.

Intracellular fluid formulation for electrophysiological experiments: 145 mM KCl, 1 mM $MgCl_2$, 5 mM EGTA, 10 mM HEPES, 5mM MgATP (adjusted to pH=7.3 with KOH).

Extracellular fluid formulation for electrophysiological experiments: 140 mM NaCl, 5mM KCl, 2 mM $CaCl_2$, 1.5 mM $MgCl_2$, 10 mM HEPES, 10 mM glucose (adjusted to pH=7.4 with NaOH).

To record the whole-cell currents of KCNQ2 and KCNQ4 channels, the cells were clamped at a holding potential of -120 mV, and then depolarized to -10 mV for a duration of 1500 ms, and the stimulation frequency was 0.1 Hz. The current amplitudes at the -10 mV test pulse before ($I_{Control}$) and after ($I_{Drug}$) the perfusion of the compounds were collected to determine the activation efficacy ($I_{Drug}$ / $I_{Control}$).

3. Compound dissolution and preparation

A certain mass of the compound was weighed and dissolved in DMSO to prepare a 20 mM DMSO stock solution which was frozen and stored at -20°C until use. On the day of the test, the 20 mM compound stock solution was diluted with extracellular fluid to the final concentration to be detected, ensuring that the DMSO content in the test drug solution did not

exceed 0.5%. This kind of concentration of DMSO has no effect on the detected KCNQ channel current. For example, to prepare 100 nM and 1 μM compound solutions, the gradient dilution method was as follows: 5 μL DMSO stock solution was first pipetted and added to 10 mL of extracellular fluid, and was dissolved evenly to obtain a 10 μM compound solution; then 1 mL of 10 μM compound solution was pipetted and added to 9 mL of extracellular fluid, dissolved evenly to obtain a 1 μM compound solution; then 1 mL of 1 μM compound was pipetted and added to 9 mL of extracellular fluid, dissolved evenly to obtain a 100 nM compound solution.

4. Experimental results and data analysis

Data collection, analysis and processing were performed using pClamp10 (Molecular Devices, Sunnyvale, CA), GraphPad Prism 5 (GraphPad Software, San Diego, CA) and Excel (Microsoft Corporation) software. All data are expressed as mean ± standard error (Mean ± SEM), significance analysis was performed using unpaired student's t-test. When $P < 0.05$, the difference between the two groups was considered statistically significant. The effect of a compound on current flow is calculated using the following formula:

$$\text{Activation efficacy} = I_{\text{Drug}} / I_{\text{Control}};$$

wherein $I_{Control}$ is the steady-state maximum value of the current peak generated in cells stimulated at a test voltage of -10 mV after being given blank extracellular fluid and $I_{Drug}$ is the steady-state maximum value of the current peak generated in cells stimulated at a test voltage of -10 mV after perfusion with a compound. When $I_{Drug} / I_{Control} > 1$, it indicates an enhancing effect, when $I_{Drug} / I_{Control} < 1$, it indicates an inhibitory effect, and when $I_{Drug} / I_{Control} = 1$, it indicates no effect. *n* is the number of cells detected.

| Compound | KCNQ4 ($I_{Drug}$ / $I_{Control}$ 100 nM) | *n* | KCNQ2 ( $I_{Drug}$ / $I_{Control}$ 100 nM) | *n* |
|---|---|---|---|---|
| K31 | 3.06 ± 0.61 | 3 | 1.07 ± 0.04 | 3 |
| K32 | 2.50 ± 0.28 | 3 | 1.05 ± 0.02 | 5 |
| K33 | 3.00 ± 0.18 | 3 | 1.06 ± 0.03 | 3 |
| K34 | 4.40 ± 0.62 | 3 | 1.07 ± 0.04 | 5 |
| K35 | 3.30 ± 0.62 | 3 | 0.98 ± 0.03 | 4 |
| K36 | 3.50 ± 0.28 | 3 | 1.05 ± 0.03 | 4 |
| K37 | 3.28 ± 0.21 | 3 | 1.02 ± 0.04 | 3 |
| K38 | 1.66 ± 0.19 | 4 | 0.96 ± 0.01 | 4 |
| K39 | 1.46 ± 0.02 | 3 | 1.10 ± 0.02 | 5 |
| K40 | 1.26 ± 0.16 | 3 | 0.97 ± 0.02 | 3 |
| K41 | 1.69 ± 0.19 | 3 | 1.03 ± 0.02 | 3 |
| K42 | 1.35 ± 0.08 | 3 | 1.02 ± 0.02 | 4 |
| K43 | 2.72 ± 0.38 | 3 | 0.98 ± 0.00 | 3 |

Results and discussion: It can be seen from the above electrophysiological experiment results that the compounds disclosed in the present invention maintain the agonistic activity of KCNQ4 potassium channel well, but all compounds are inactive towards KCNQ2, showing good selectivity.

The above embodiments are only used to illustrate the technical solution of the present invention, but not to limit it. Although the present invention has been described in detail with reference to the foregoing embodiments, those of ordinary skilled in the art will understand that the technical solutions described in the foregoing embodiments can be modified, or equivalent substitutions can be made for some or all of the technical features, without departing from the spirit and essence of the invention as defined by the claims; and these modifications or substitutions are still within the scope of the claims of the present invention.

## Claims

1. A compound represented by formula I or a pharmaceutically acceptable salt thereof,

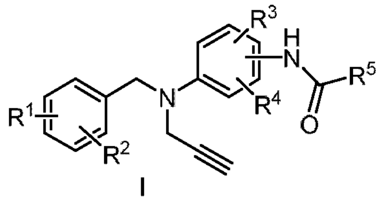

I

wherein:

$R^1$ and $R^2$ are each independently selected from the group consisting of hydrogen, halogen, $C_1$-$C_6$ alkoxy, halogenated $C_1$-$C_6$ alkoxy, $C_1$-$C_6$ alkylthio, $C_4$-$C_6$ tert-alkyl, halogenated $C_1$-$C_6$ alkyl, nitro and cyano;
$R^3$ and $R^4$ are each independently selected from the group consisting of hydrogen, halogen and $C_1$-$C_6$ alkyl;
$R^5$ is selected from the group consisting of $C_1$-$C_6$ alkyl, $C_1$-$C_3$ alkoxy, $C_4$-$C_{14}$ tert-alkoxy, phenoxy and benzyloxy; preferably, $R^5$ is selected from the group consisting of $C_1$-$C_3$ alkoxy, $C_4$-$C_6$ tert-alkoxy, phenoxy and benzyloxy; more preferably, R5 is selected from the group consisting of methoxy, ethoxy, isopropoxy, tert-butoxy, phenoxy and benzyloxy; and R5 is most preferably tert-butoxy.

2. The compound or the pharmaceutically acceptable salt thereof according to claim 1, wherein $R^1$ and $R^2$ are each independently selected from the group consisting of halogen, $C_1$-$C_6$ alkoxy, halogenated $C_1$-$C_6$ alkoxy, $C_1$- $C_6$ alkylthio, tert-butyl, nitro, cyano and trifluoromethyl;
preferably, $R^3$ and $R^4$ are each independently hydrogen.

3. The compound or the pharmaceutically acceptable salt thereof according to claim 1, wherein the compound of formula I is a compound of formula II below:

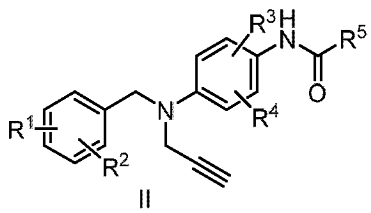

II

wherein the definitions of $R^1$, $R^2$, $R^3$, $R^4$ and $R^5$ are the same as those in the corresponding claim.

4. The compound or the pharmaceutically acceptable salt thereof according to claim 1, wherein the compound of formula I is a compound of formula III below:

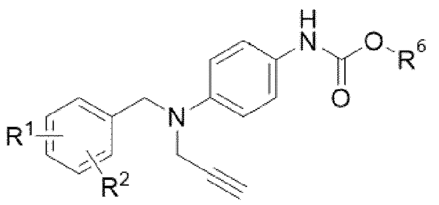

III

wherein the definitions of $R^1$ and $R^2$ are the same as those in the corresponding claim;
$R^6$ is $C_4$-$C_6$ tert-alkyl;
preferably, the compound of formula I is a compound of formula c below:

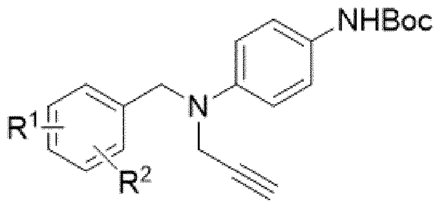

c

wherein the definitions of $R^1$ and $R^2$ are the same as those in the corresponding claim.

**5.** The compound or the pharmaceutically acceptable salt thereof according to claim 1, wherein the compound of formula I is selected from the following compounds:

| Compound Number | Compound Structure |
|---|---|
| K31 | 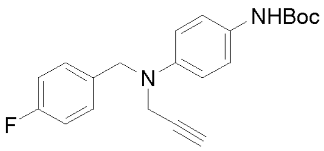 |
| K32 | 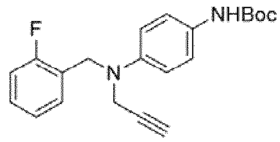 |
| K33 | 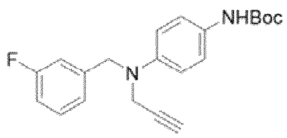 |
| K34 | 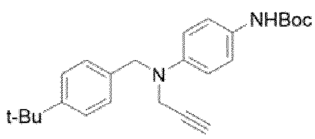 |
| K35 | 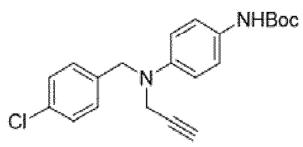 |
| K36 | 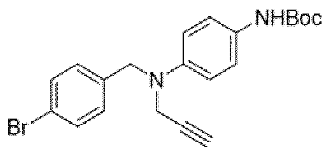 |
| K37 | 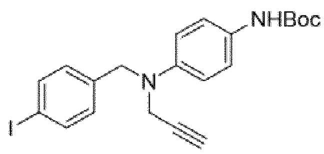 |
| K38 | 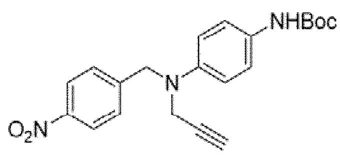 |

(continued)

| Compound Number | Compound Structure |
|---|---|
| K39 | 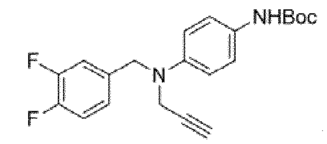 |
| K40 | 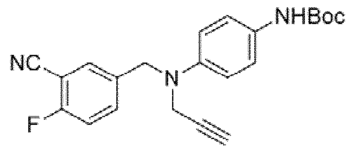 |
| K41 | 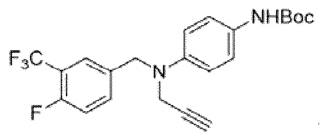 |
| K42 | 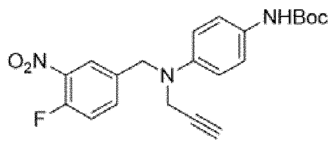 |
| K43 | 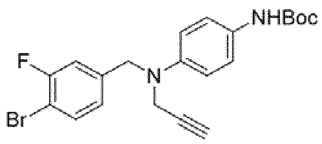 |

**6.** A method for preparing the compound according to any one of claims 1 to 5, wherein the compound is a compound of formula c, and the method includes steps of:

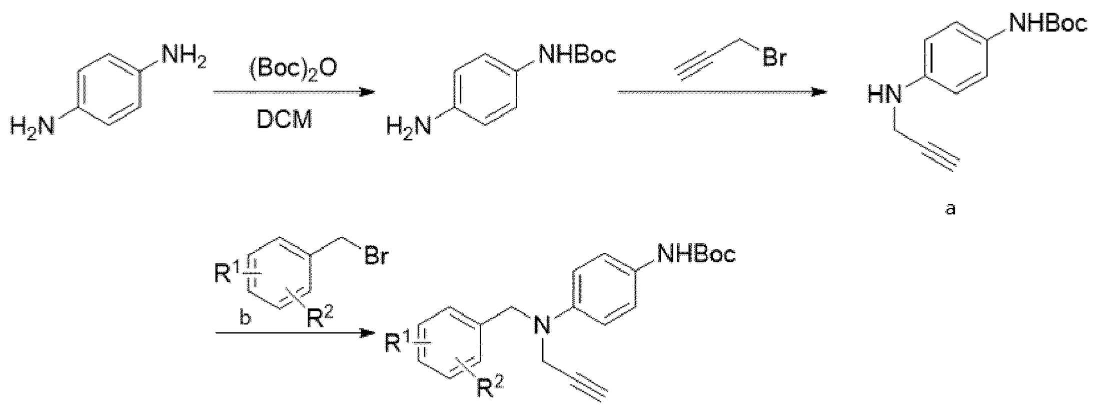


a

c

reacting 1,4-phenylenediamine with di-tert-butyl dicarbonate to produce N-(tert-butoxycarbonyl)-1,4-phenylenediamine;
subjecting N-(tert-butoxycarbonyl)-1,4-phenylenediamine and bromopropyne to a substitution reaction to obtain intermediate a; and
reacting the intermediate a with compound b to produce compound of formula c;
wherein the definitions of $R^1$ and $R^2$ are the same as those in the corresponding claims respectively.

**7.** A pharmaceutical composition comprising the compound or the pharmaceutically acceptable salt thereof according to any one of claims 1 to 5 and optionally, a pharmaceutically acceptable excipient.

8. Use of the compound or the pharmaceutically acceptable salt thereof according to any one of claims 1 to 5, or the pharmaceutical composition according to claim 7 in preparation of a KCNQ4 potassium channel agonist.

9. Use of the compound or the pharmaceutically acceptable salt thereof according to any one of claims 1 to 5, or the pharmaceutical composition according to claim 7 in preparation of a medicament for treating a disease associated with smooth muscle or skeletal muscle.

10. The use according to claim 9, wherein the disease associated with smooth muscle or skeletal muscle includes visceral pain, indigestion, irritable bowel syndrome, overactive bladder syndrome, hypertension, pulmonary hypertension, coronary artery disease, cerebral vasospasm, asthma, chronic obstructive pulmonary disease, prenatal labor pain, pruritus, sexual dysfunction and deafness.

**INTERNATIONAL SEARCH REPORT**

International application No. **PCT/CN2023/071587**

**A. CLASSIFICATION OF SUBJECT MATTER**

C07C271/28(2006.01)i;C07C269/04(2006.01)i;C07C269/06(2006.01)i;A61K31/27(2006.01)i;A61K31/277(2006.01)i; A61P19/00(2006.01)i;A61P1/14(2006.01)i;A61P13/10(2006.01)i;A61P9/12(2006.01)i;A61P11/00(2006.01)i;A61P11/06(2006.01)i;A61P15/00(2006.01)i;A61P17/04(2006.01)i;A61P27/16(2006.01)i;A61P25/00(2006.01)i

According to International Patent Classification (IPC) or to both national classification and IPC

**B. FIELDS SEARCHED**

Minimum documentation searched (classification system followed by classification symbols)

IPC: C07C A61K A61P

Documentation searched other than minimum documentation to the extent that such documents are included in the fields searched

Electronic data base consulted during the international search (name of data base and, where practicable, search terms used)

CNTXT; CNKI; VEN; WOTXT; EPTXT; USTXT; STN; 万方, WANFANG: 中国科学院上海药物研究所, 南发俊, 高召兵, 董武恒, 郑月明, 王锦涛, 周晓宇, 刘桦楠, 许海燕, 瑞替加滨, 衍生物, 钾离子通道, 激动剂, 骨骼肌, 平滑肌, Retigabine, derivative, K+ channel, KCNQ4, Kv7, agonist, smooth, skeletal, muscle, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| X | CN 103073455 A (SHANGHAI INSTITUTE OF MATERIA MEDICA, CHINESE ACADEMY OF SCIENCES) 01 May 2013 (2013-05-01) claims 1-9 | 1-3, 7 |
| A | CN 103073455 A (SHANGHAI INSTITUTE OF MATERIA MEDICA, CHINESE ACADEMY OF SCIENCES) 01 May 2013 (2013-05-01) claims 1-9 | 4-6, 8-10 |
| X | CN 105017085 A (SHANGHAI INSTITUTE OF MATERIA MEDICA, CHINESE ACADEMY OF SCIENCES) 04 November 2015 (2015-11-04) claims 5-7 | 1, 2, 7 |
| A | CN 105017085 A (SHANGHAI INSTITUTE OF MATERIA MEDICA, CHINESE ACADEMY OF SCIENCES) 04 November 2015 (2015-11-04) claims 5-7 | 3-6, 8-10 |
| X | CN 109641836 A (SCIFLUOR LIFE SCIENCES INC.) 16 April 2019 (2019-04-16) description, paragraphs [0232]-[0235] | 1, 2, 7 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 February 2023** | **06 April 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2023/071587**

**C. DOCUMENTS CONSIDERED TO BE RELEVANT**

| Category* | Citation of document, with indication, where appropriate, of the relevant passages | Relevant to claim No. |
|---|---|---|
| A | CN 109641836 A (SCIFLUOR LIFE SCIENCES INC.) 16 April 2019 (2019-04-16)<br>description, paragraphs [0232]-[0235] | 3-6, 8-10 |
| A | WANG LEI et al. "Discovery of Novel Retigabine Derivatives as Potent KCNQ4 and KCNQ5 Channel Agonists with Improved Specificity"<br>*ACS Medicinal Chemistry Letters,* Vol. 10, No. 1, 19 December 2018 (2018-12-19), ISSN: 1948-5875,<br>pages 27-33 | 1-10 |

Form PCT/ISA/210 (second sheet) (July 2022)

**INTERNATIONAL SEARCH REPORT**
**Information on patent family members**

International application No.
**PCT/CN2023/071587**

| Patent document cited in search report | Publication date (day/month/year) | Patent family member(s) | Publication date (day/month/year) |
|---|---|---|---|
| CN 103073455 A | 01 May 2013 | US 2014336252 A1 | 13 November 2014 |
| | | US 9353048 B2 | 31 May 2016 |
| | | KR 20140090643 A | 17 July 2014 |
| | | KR 101697834 B1 | 19 January 2017 |
| | | JP 2014532629 A | 08 December 2014 |
| | | JP 5947907 B2 | 06 July 2016 |
| | | WO 2013060097 A1 | 02 May 2013 |
| | | EP 2772481 A1 | 03 September 2014 |
| | | EP 2772481 A8 | 26 November 2014 |
| | | EP 2772481 A4 | 27 May 2015 |
| | | EP 2772481 B1 | 18 January 2017 |
| | | CN 103073455 B | 19 August 2015 |
| | | ID 201503664 A | 21 August 2015 |
| | | SG 11201401759 B | 03 August 2016 |
| | | HK 1200437 A1 | 20 October 2017 |
| | | IN 324738 B | 15 November 2019 |
| | | SG 11201401759 A1 | 26 September 2014 |
| | | IN 201401113 P2 | 16 October 2015 |
| | | HK 1200437 A0 | 07 August 2015 |
| CN 105017085 A | 04 November 2015 | EP 3138833 A1 | 08 March 2017 |
| | | EP 3138833 A4 | 26 April 2017 |
| | | EP 3138833 B1 | 03 November 2021 |
| | | WO 2015165352 A1 | 05 November 2015 |
| | | KR 20160147278 A | 22 December 2016 |
| | | KR 101881983 B1 | 25 July 2018 |
| | | CA 2947329 A1 | 05 November 2015 |
| | | CA 2947329 C | 02 October 2018 |
| | | US 2017081301 A1 | 23 March 2017 |
| | | US 10077245 B2 | 18 September 2018 |
| | | JP 2017518274 A | 06 July 2017 |
| | | JP 6357248 B2 | 11 July 2018 |
| | | AU 2015252640 A1 | 24 November 2016 |
| | | AU 2015252640 B2 | 21 September 2017 |
| | | US 2018134677 A1 | 17 May 2018 |
| | | US 10316008 B2 | 11 June 2019 |
| | | CN 105017085 B | 29 June 2018 |
| CN 109641836 A | 16 April 2019 | US 2017355679 A1 | 14 December 2017 |
| | | KR 20190008411 A | 23 January 2019 |
| | | AU 2017278093 A1 | 22 November 2018 |
| | | AU 2017278093 B2 | 16 December 2021 |
| | | AU 2017278093 B8 | 23 December 2021 |
| | | EP 3468947 A1 | 17 April 2019 |
| | | EP 3468947 A4 | 08 January 2020 |
| | | US 2021309612 A1 | 07 October 2021 |
| | | JP 2019518754 A | 04 July 2019 |
| | | WO 2017214539 A1 | 14 December 2017 |
| | | MX 2018015251 A | 25 April 2019 |
| | | CA 3023340 A1 | 14 December 2017 |
| | | RU 2018147263 A | 10 July 2020 |
| | | BR 112018075597 A2 | 26 March 2019 |

Form PCT/ISA/210 (patent family annex) (July 2022)

**INTERNATIONAL SEARCH REPORT**
**Information on patent family members**

International application No.
**PCT/CN2023/071587**

| Patent document cited in search report | Publication date (day/month/year) | Patent family member(s) | Publication date (day/month/year) |
|---|---|---|---|
| | | IN 201817045411 A | 11 October 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5384330 A
- WO 2005087754 A
- WO 2008024398 A
- WO 2013060097 A
- CN 105017085 A


### Non-patent literature cited in the description


- **BROWN et al.** *Br J Pharmacol.*, 2009, vol. 156 (8), 1185-1195
- **STOTT et al.** *Drug Discov Today.*, 2014, vol. 19 (4), 413-424
- **JENTSCH**. *Nat Rev Neurosci.*, 2000, vol. 1 (1), 21-30
- **DU et al.** *Br J Pharmacol.*, 2018, vol. 175 (12), 2158-2172
- **SZELENYI**. *Inflamm Res.*, 2013, vol. 62 (3), 251-258
- **YEUNG et al.** *Br J Pharmacol.*, 2007, vol. 151 (6), 758-770
- **IPAVEC et al.** *Pharmacol Res.*, 2011, vol. 64 (4), 397-409
- **EVSEEV et al.** *Front Physiol.*, 2013, vol. 4, 277
- **NG et al.** *Br J Pharmacol.*, 2011, vol. 162 (1), 42-53
- **TERRENOIRE et al.** *Circ Res*, 2005, vol. 96 (5), e25-34
- **ORHAN et al.** *Expert Opin Pharmacother.*, 2012, vol. 13 (12), 1807-16
- **KUMAR et al.** *Mol Pharmacol.*, 2016, vol. 89 (6), 667-677
- **ZHANG et al.** *Br J Pharmacol.*, 2016, vol. 173 (24), 3480-3491
- **HAICK et al.** *Pharmacol Ther.*, 2016, vol. 165, 14-25
- **BARRESE et al.** *Annu Rev Pharmacol Toxicol.*, 2018, vol. 58, 625-648
- **S. JANKOVIC et al.** *Expert Opinion on Drug Discovery*, 2013, vol. 8 (11), 1-9
- **F. RODE et al.** *European Journal of Pharmacology*, 2010, vol. 638, 121-127
- **KUMAR et al.** *Mol Pharmacol.*, 2016, vol. 89 (6), 667-77